# EUROPEAN PATENT SPECIFICATION

(11) **EP 2 216 325 B1**
(45) Date of publication and mention of the grant of the patent: **14.01.2015**
(21) Application number: 08846952.3
(22) Date of filing: 04.11.2008
(51) Int. Cl.: C07C 303/08, C07C 309/80, C07C 303/22

(54) **PROCESS FOR PREPARATION OF TRIFLUOROMETHANESULFONYL FLUORIDE**
VERFAHREN ZUR HERSTELLUNG VON TRIFLUOROMETHANSULFONYLFLUORID
PROCÉDÉ DE PRÉPARATION DE FLUORURE DE TRIFLUOROMÉTHANESULFONYLE

(30) Priority: 06.11.2007 JP 2007287969; 31.10.2008 JP 2008280872
(43) Date of publication of application: 11.08.2010
(73) Proprietor: Central Glass Company, Limited, Yamaguchi 755-0001 (JP)
(72) Inventor: SAKAI, Shigenori, Ube-shi Yamaguchi 755-0001 (JP); MORINAKA, Takayoshi, Ube-shi Yamaguchi 755-0001 (JP); NANMYO, Tsutomu, Ube-shi Yamaguchi 755-0001 (JP); KUME, Takashi, Ube-shi Yamaguchi 755-0001 (JP)
(74) Representative: Manitz, Finsterwald & Partner GbR
(86) International application number: PCT/JP2008/070016
(87) International publication number: WO 2009/060815

(56) References cited:
- JP-A- 6 263 715
- JP-A- 51 125 322
- US-A- 2 732 398
- US-A- 5 540 818
- IL'CHENKO A Y ED - BELLUS D (ED): "Tetrahetero-substituted methanes with a carbon-halogen bond", 1 January 2005 (2005-01-01), SCIENCE OF SYNTHESIS. CATEGORY 3 : COMPOUNDS WITH FOUR AND THREE CARBON-HETEROATOM BONDS; [METHODS OF MOLECULAR TRANSFORMATIONS. (HOUBEN-WEYL)], STUTTGART : GEORG THIEME VERLAG, DE, PAGE(S) 1135 - 1201, XP008106589, ISBN: 978-3-13-118681-2 * Method 15; page 1165 *

## Description

### Technical Field

The present invention relates to a process for producing trifluoromethanesulfonyl fluoride (CF₃SO₂F), which is useful as a fluorination reagent and as a raw material for intermediates in the fields of organic syntheses, pharmaceutical and agrichemical products and electronic materials.

### Background Art

Conventionally, electrochemical fluorination is known as one technique for production of fluoroalkanesulfonyl fluorides including trifluoromethanesulfonyl fluoride. For example, Patent Document 1 discloses a fluoride production process using an electrochemical fluorination reaction of methanesulfonyl chloride (CH₃SO₂Cl) in hydrogen fluoride anhydride. This process produces a mixed gas of about 20 vol% trifluoromethanesulfonyl fluoride and about 80 vol% hydrogen in an electrolytic cell. It is thus necessary to isolate and purify the target product to a high purity from such a large volume of hydrogen. It is also necessary to provide large-scale electrolytic reaction equipment for the high-level isolation and purification, which results in a high cost problem.

Against the above backdrop, there have been proposed some fluoroalkanesulfonyl fluoride production processes that do not use the electrochemical fluorination technique. For example, Patent Document 2 discloses a process for producing a perfluoroalkanesulfonyl fluoride (Rf-CF₂-SO₂F) by reacting a perfluoroolefin as a starting material with sulfuric anhydride, hydrolyzing the resulting perfluoroalkane sultone to a monohydroperfluoroalkanesulfonyl fluoride (Rf-CHF-SO₂F), and then, reacting the monohydroperfluoroalkanesulfonyl fluoride with fluorine or fluorine-containing gas. Patent Document 3 discloses a process for producing a perfluoroalkanesulfonyl fluoride or hydrofluoroalkanesulfonyl fluoride by reacting an alkanesulfonyl fluoride with a fluorine-containing gas.

On the other hand, Non-Patent Document 1 discloses a process for producing pentafluoroethanesulfonyl fluoride (C₂F₅SO₂F) by reaction of pentafluoroethanesulfonyl chloride (C₂F₅SO₂Cl) with cesium fluoride. Patent Document 4 discloses a process for producing a methanesulfonyl fluoride derivative by reacting a corresponding methanesulfonyl chloride derivative with sodium fluoride, potassium fluoride or cesium fluoride in anhydrous acetonitrile in the presence of a crown ether. Patent Document 5 discloses a process for producing methanesulfonyl fluoride by reaction of methanesulfonyl chloride (CH₃SO₂Cl) with a fluoride and water. Patent Document 6 discloses a process for producing a sulfonic fluoride by reaction of a sulfonic halide with an inorganic fluoride in an organic/aqueous phase in the presence of a catalytic amount of amine or onium salt.

Further, Patent Document 7 discloses a process for producing trifluoromethanesulfonyl fluoride by using the same starting material as that of the present invention in the coexistence of a quaternary halide salt in the reaction system.
Patent Document 1: U.S. Patent No. 2732398
Patent Document 2: International Publication No. WO2004/096759
Patent Document 3: Japanese Laid-Open Patent Publication No. 2003-206272
Patent Document 4: Japanese Laid-Open Patent Publication No. 6-072987
Patent Document 5: Japanese Laid-Open Patent Publication No. 51-125322

Non-Patent Document 1: O. A. Radchenko, A. Ya. Il'chenko and L. M. Yagupol'skii, Zh. Org. Khim., Vol. 15, 500-503 (1981)

### Disclosure of the Invention

In the process of Patent Document 1 and Patent Document 2 (electrolytic fluorination process), the mixed gas of about 20 vol% trifluoromethanesulfonyl fluoride and about 80 vol% hydrogen is produced from the reaction solution in the electrolytic cell during the reaction. In the coexistence of 0.1 % to 0.2% of oxidizable gas substance, it is inevitable to handle the combustible gas in order to isolate and purify the trifluoromethanesulfonyl fluoride to a high purity from the hydrogen-mixed reaction solution. This process thus requires large-scale production equipment (high-pressure combustible gas equipment) and entails danger.

Further, the target product becomes harder to isolate and purify in the coexistence of many by-products of the electrolytic fluorination, other than the hydrogen gas, (such as monofluoromethanesulfonyl fluoride, difluoromethanesulfonyl fluoride etc.) in the reaction system. In view of the fact that, for example, even several ppm of impurities would provide a large influence on electronic equipment in the field of electronic materials, it is difficult by this process to produce the trifluoromethanesulfonyl fluoride with high purity.

The process of Patent Document 2, which does not uses the electrolytic fluorination technique, favorably gives a high target product yield of 88% but becomes complicated with the need for multiple process steps. This process is thus difficult to use for industrial production applications. The process of Patent Document 3 is also difficult to use for industrial applications because of a very low production yield of the perfluoroalkanesulfonyl fluoride.

As is conventionally known, a reaction of methanesulfonyl chloride with a metal fluoride in the presence of water forms a corresponding fluoride (CH₃SO₂Cl + KF → CH₃SO₂F + KCl). The process of Patent Document 5 uses such a reaction as mentioned above.

In the case of applying the process of Patent Document 5 to fluorinate trifluoromethanesulfonyl chloride, which is the starting material of the present invention, however, the target fluoride compound can be obtained with very low production yield (∼3%). This process is thus difficult to use for industrial production of the trifluoromethanesulfonyl fluoride.

In consequence, the conventional processes are not always satisfied in view of the reaction yield, the ease of reaction control, the product purity, the process complexity and the like. It has been desired to develop a process for producing trifluoromethanesulfonyl fluoride easily with high yield and high purity on an industrial scale.

As a result of extensive researches to solve the prior art problems, the present inventors have found that it is possible to produce trifluoromethanesulfonyl fluoride as a target compound with high yield and high selectivity by reacting trifluoromethanesulfonyl chloride (CF₃SO₂Cl) with a metal fluoride in the presence of water while controlling the amount of the water to within the range of 0.6 mass% to 10.0 mass% based on 100 mass% of the metal fluoride. The present invention is based on such a finding.

The present invention is characterized by the amount of the water used. When the amount of the water is less than 0.6 mass% based on 100 mass% of the metal fluoride, the reaction can hardly proceed. (See Comparative Example 1 mentioned later.) When the amount of the water exceeds 10.0 mass% based on 100 mass% of the metal fluoride, there readily occurs a side reaction in which the trifluoromethanesulfonyl chloride itself undergoes hydrolysis to form a sulfonic acid as a by-product and thereby cause a significant deterioration in target product yield so that the target trifluoromethanesulfonyl fluoride can hardly be obtained. (See Comparative Example 2 mentioned later.)

As mentioned above, the present inventors have found that it is possible to promote the reaction dramatically and obtain the target product with high yield and high selectivity and with almost no side reaction product by controlling the amount of the water to within the range of 0.6 mass% to 10 mass% based on 100 mass% of the metal fluoride.

The reason for this is assumed that the starting material of the present invention, i.e., trifluoromethanesulfonyl chloride shows different reactivity from the methanesulfonyl chloride under the strong electron attracting effect of a trifluoromethyl group.

In Patent Document 7, the trifluoromethanesulfonyl fluoride is produced in the coexistence of the quaternary halide salt in the reaction system as mentioned above. In the present invention, by contrast, the trifluoromethanesulfonyl fluolide can be produced with high yield, without the need to use any quaternary halide salt, by controlling the water amount to within the above specific range. The present invention enables production of the target trifluoromethanesulfonyl fluoride with high productivity and has no industrial load and no need for purification.

### Detailed Description

The present invention provides a process for producing trifluoromethanesulfonyl fluoride by a very simplified procedure with high reaction yield, ease of reaction control and satisfactory product purity as compared with the conventional processes.

Hereinafter, the present invention will be described in detail below. The starting material of the present invention, i.e., trifluoromethanesulfonyl chloride can be easily prepared by any known technique as disclosed in e.g. Japanese Patent No. 3456634, Japanese Patent No. 3444477 or Japanese Laid-Open Patent Publication No. 2000-264871.

The metal halide used in the present invention is a fluoride of an alkali metal such as lithium (Li), sodium (Na), potassium (K), rubidium (Rb) or cesium (Cs).

Specific examples of the metal fluoride are lithium fluoride, sodium fluoride, potassium fluoride, rubidium fluoride and cesium fluoride. Among others, preferred are sodium fluoride, potassium fluoride and cesium fluoride because of their easy availability. These fluorides can be used solely or in the form of a mixture of two or more thereof.

The amount of the metal fluoride used is generally 1 to 10 moles, preferably 1 to 8 moles, more preferably 1.5 to 6 moles, per 1 mole of the trifluoromethanesulfonyl chloride. When the amount of the metal fluoride is less than 1 mole, it becomes a cause of deterioration in reaction yield. When the amount of the metal fluoride exceeds 10 moles, the reaction proceeds with no problem. In this case, however, there is no merit in terms of the reaction rate and reaction yield. It is further economically unfavorable to use such a large amount of the metal fluoride.

In the present invention, the specific amount of the water is preferably used as a reaction solvent. The amount of the water used is generally in the range of 0.6 mass% to 10.0 mass% based on 100 mass% of the metal fluoride. In particular, the amount of the water is preferably in the range of 0.6 mass% to 8.0 mass%, more preferably 0.8 mass% to 6.0 mass%.

It has been further found that it is possible to obtain the target product with higher yield and higher selectivity by not only controlling the amount of the water to within the range of 0.6 mass% to 10.0 mass% based on 100 mass% of the metal fluoride but also controlling the reaction temperature to 20°C or higher. It is particularly preferable to perform the reaction at a temperature of 20 to 70°C for ease of temperature control.

Although the reaction proceeds even at a temperature lower than 20°C, the water in the reaction system may be frozen depending on the temperature (e.g. at around 0°C). There is thus less merit in performing the reaction at such a temperature lower than 20°C.

Further, there may occur a side reaction to form a sulfonic acid concurrently with the production reaction of the trifluoromethanesulfonyl fluoride when the reaction temperature becomes higher than necessity. The reaction temperature can be controlled by a concerned party to an appropriate level that does not cause industrial production load.

As a particularly preferred embodiment of the present invention, it is feasible to control the water amount and reaction temperature to within the respective favorable ranges, e.g., to control the amount of the water to 1.0 mass% based on 100 mass% of the metal fluoride and control the temperature of the reaction system to 40 to 50°C as in Example 1 mentioned later, so as to promote the reaction dramatically and obtain the target product with high yield and high selectivity and with almost no by-product.

Although the water is used as the reaction solvent, an organic solvent may be used in combination with the water as the reaction solvent. Herein, the organic solvent refers to an inert organic compound that is not directly involved in the reaction of the present invention. Specific examples of the organic solvent are any available organic solvents such as benzene, toluene, xylene, pentane, hexane, heptane, acetonitrile, carbon tetrachloride, chloroform, methylene chloride, 1,2-dichloroethane, ethylbenzene, mesitylene, dioxane, dimethyl ether, diethyl ether, dibutyl ether and tetrahydrofuran. These organic solvents can be used solely or in combination of two or more thereof.

In view of the industrial application of the present invention, however, there is less merit in the coexistence of another organic solvent with the water since the reaction can proceeds sufficiently to produce the target compound with high yield and high selectivity under the specific reaction condition by the use of only the water as the reaction solvent. (See the after-mentioned examples.)

In the production process of the present invention, the reaction can be performed by e.g. adding the metal fluoride in the presence of the water, stirring the metal fluoride with the water, and then, adding the trifluoromethanesulfonyl chloride to the mixed solution. There is no particular restriction on the order of addition of the reaction reagents. It is however preferable in the present invention to add the trimethalsulfonyl chloride into the reaction system after the metal fluoride and the water in terms of the production efficiency.

There is also no particular restriction on the reaction time. It is preferable to finish the reaction after confirming by chromatography etc. that the raw materials have been consumed sufficiently so that the reaction would no longer proceed. The reaction time can be selected as appropriate by a concerned party. It is further preferable to perform the reaction with stirring for good reaction efficiency.

The reaction proceeds regardless of whether to add the trifluoromethanesulfonyl chloride into the reaction system at a time or continuously. Although the addition technique can be selected as appropriate by a concerned party, it is preferable to continuously drop the trifluoromethanesulfonyl chloride into the reaction system. It is also preferable to finish dropping the trifluoromethanesulfonyl chloride for about 1 hour to 4 hours.

Furthermore, the reaction can be performed under pressurized condition in the present invention. The target product of the present invention, i.e., trifluoromethanesulfonyl fluoride has a very low boiling point (-23°C) and exists as a gas at room temperature. In the present invention, the trifluoromethanesulfonyl fluoride is generated in the reaction system when the reaction is performed in the above temperature range. It is preferable to perform the reaction under the pressurized conditions in the reaction system closed using any pressure-resistant reactor as the trifluoromethanesulfonyl fluoride is in gaseous form in the reaction temperature range of 20 to 70°C.

In the case of performing the reaction under the pressurized conditions, the reaction pressure is generally controlled to 0.1 to 10 MPa, preferably 0.1 to 5 MPa, more preferably 0.3 to 2 MPa, (as absolute pressure, the same applies to the following).

There is no particular restriction on the material of the reactor as long as it is resistant to the reaction pressure. As the reactor, there can be used a reaction vessel having a lining of tetrafluoroethylene resin, chlorotrifluoroethylene resin, vinylidene fluoride resin, PFA resin, glass etc. or a glass reaction vessel. A metal reaction vessel of stainless steel, Hastelloy, Monel etc. can alternatively be used as the reactor.

It has also been found that it is possible to obtain the target product of the present invention, trifluoromethanesulfonyl fluoride, easily with high purity and without the need for any distillation operation, by passing the reaction product gas through a condenser while cooling the condenser down to a temperature lower than 0°C, more specifically -20 to -30°C, and then, collecting the resulting gas as the trifluoromethanesulfonyl fluoride has a very low boiling point (-23°C) and exists in gaseous form at room temperature.

For example, the following procedure is conceivable. The water, potassium fluoride and trifluoromethanesulfonyl chloride are added into the reactor, followed by passing the resulting gas through the condenser cooled to -30°C and refluxing the condensed gas into the reactor while heating the reaction solution to room temperature. Under the above reflux operation, the condenser is partly opened to collect a part of the condensed gas by a trap. After the completion of the reaction, the condenser is heated to about -20°C to thereby collect all of the remainder of the gas by the trap. In this way, the trifluoromethanesulfonyl fluoride can be obtained with high purity and without the need for any distillation operation. (See the after-mentioned examples.) The need for no distillation operation is one favorable feature of the present invention.

The process of the present invention can be performed in a continuous manner, a semi-continuous manner or a batch manner. The manner of the reaction process can be selected as appropriate by a concerned party.

As discussed above, it is possible in the present invention to obtain the trifluoromethanesulfonyl fluoride with high purity by the simple procedure, without the mixing of water or trifluoromethanesulfonyl chloride into the product.

The present invention will be described in more detail below by way of the following examples. It should be noted that these examples are illustrative and are not intended to limit the present invention thereto. Herein, the term "%" of a composition analysis value represents "area%" of an organic component in a reaction mixture as measured by gas chromatography (using a flame ionization detector FID, unless otherwise specified).

### Example 1

A 500-mL pressure-resistant reactor was charged with 103 g (1.78 mol) of 1% water-containing potassium fluoride, cooled to 5°C, and then, vacuumed. After that, trifluoromethanesulfonyl chloride (100 g (0.595 mol)) was added into the reactor. The reactor was sealed and gradually heated. The resulting solution was stirred for 4 hours under the conditions that the inside temperature and pressure of the reactor were controlled to 40 to 50°C and 0.94 MPa, respectively. The gas inside the reactor was fed through a condenser under cooling at -40°C to -20°C and collected by a trap under liquid nitrogen cooling. The collected product weighed 80.3 g and was confirmed by gas chromatography (GC) to be trifluoromethanesulfonyl fluoride with a purity of 98.9% and a yield of 88% (conversion rate: 99%, selectivity: 89%).
(It is herein noted that, in Example 1, the term "1% water-containing fluoride hydrate" means a potassium fluoride hydrate containing 1% of water based on 100% of potassium fluoride; and the same applies to the following.)

### Example 2

A 500-mL pressure-resistant reactor was charged with 103 g (1.78 mol) of 3% water-containing potassium fluoride, cooled to 5°C, and then, vacuumed. After that, trifluoromethanesulfonyl chloride (100 g (0.595 mol)) was added into the reactor. The reactor was sealed and gradually heated. The resulting solution was stirred for 7 hours under the conditions that the inside temperature and pressure of the reactor were controlled to 40 to 50°C and 0.90 MPa, respectively. The gas inside the reactor was fed through a condenser under cooling at -40°C to -20°C and collected by a trap under liquid nitrogen cooling. The collected product weighed 54.2 g and was confirmed by gas chromatography (GC) to be trifluoromethanesulfonyl fluoride with a purity of 98.8% and a yield of 81 % (conversion rate: 99%, selectivity: 81 %).

It has been shown by the above experiments that the reaction can proceed favorably and produce the target trifluoromethanesulfonyl fluoride with high yield and high selectivity when the amount of the water is in the range of 0.6 mass% to 10.0 mass% based on 100 mass% of the metal fluoride.

### [Comparative Example 1]

A 500-mL pressure-resistant reactor was charged with 103 g (1.78 mol) of 0.2% water-containing potassium fluoride, cooled to 5°C, and then, vacuumed. After that, trifluoromethanesulfonyl chloride (100 g (0.595 mol)) was added into the reactor. The reactor was sealed and gradually heated. The resulting solution was stirred for 22 hours under the conditions that the inside temperature and pressure of the reactor were controlled to 40 to 50°C and 0.24 MPa, respectively. The gas inside the reactor was fed through a condenser under cooling at -40°C to -20°C and collected by a trap under liquid nitrogen cooling. The collected product weighed 5.3 g and was confirmed by gas chromatography (GC) to be trifluoromethanesulfonyl fluoride with a purity of 74.7% and a yield of 4% (conversion rate: 5%, selectivity: 97%).

It has been shown that the reaction cannot proceed and can yield almost no target trifluoromethanesulfonyl fluoride when the amount of the water is less than 0.6 mass% based on 100 mass% of the metal fluoride.

### [Comparative Example 2]

A 200-ml glass flask with a condenser cooled to about -30°C was charged with 100 g of water and 51.7 g (0.890 mol) of potassium fluoride. The water and the potassium fluoride were mixed well together with stirring. The resulting mixed solution was cooled to 10°C under atmospheric pressure, followed by dropping thereto 50.0 g (0.297 mol) of trifluoromethanesulfonyl chloride. After the completion of the dropping, the resulting solution was stirred for 1 hour at room temperature. The condenser was then heated to about -20°C. The reaction gas was collected by a trap under liquid nitrogen cooling. The collected product weighted 5.3g and was confirmed by gas chromatography (GC) to be trifluoromethanesulfonyl fluoride with a purity of 1.0% and a yield of 0.1%.

It has been shown that the reaction can be performed even under atmospheric pressure, but hardly proceed, when the amount of the water exceeds 10 mass% based on 100 mass% of the metal fluoride.

The details of Examples and Comparative Examples are indicated in TABLE 1.

**[TABLE 1]**

| CF₃SO₂Cl + KF + H₂O → CF₃SO₂F | | | | | | |
|---|---|---|---|---|---|---|
| | H₂O (mass%) | KF (g) | Temperature (°C) | Pressure (MPa) | Purity (%) | Yield (%) |
| Example 1 | 1.0 | 103.0 | 40-50 | 0.94 | 98.9 | 88.0 |
| Example 2 | 3.0 | 103.0 | 40-50 | 0.90 | 98.8 | 81.0 |
| Comparative Example 1 | 0.2 | 103.0 | 40-50 | 0.24 | 74.7 | 4.0 |
| Comparative Example 2 | excessive (100 g) | 51.7 | room temperature | 0.1 | 1.0 | 0.1 |

## Claims

1. A process for producing trifluoromethanesulfonyl fluoride (CF₃SO₂F), comprising: performing a reaction of trifluoromethanesulfonyl chloride (CF₃SO₂Cl) with a metal fluoride in the presence of water,
wherein the amount of the water is in the range of 0.6 mass% to 10.0 mass% based on 100 mass% of the metal fluoride.

2. The process according to claim 1, wherein the metal fluoride is at least one selected from the group consisting of lithium fluoride, sodium fluoride, potassium fluoride, rubidium fluoride and cesium fluoride.

3. The process according to claim 1 or 2, wherein the amount of the metal fluoride is 1 to 10 moles per 1 mol of the trifluoromethanesulfonyl chloride.

4. The process according to any one of claims 1 to 3, wherein the temperature of the reaction is 20°C or higher.

5. The process according to any one of claims 1 to 4, wherein the temperature of the reaction temperature is 40 to 50°C.

6. The process according to any one of claims 1 to 5, wherein the amount of the water is in the range of 1.0 mass% to 3.0 mass% based on 100 mass%.

7. The process according to any one of claims 1 to 6, wherein the reaction is performed by adding the trifluoromethanesulfonyl chloride to a hydrate of the metal fluoride.

8. The process according to any one of claims 1 to 7, wherein the reaction is performed at 20 to 70°C under pressurized conditions.

9. The process according to any one of claims 1 to 8, wherein the metal fluoride is at least one selected from the group consisting of sodium fluoride, potassium fluoride and cesium fluoride.

10. A process for producing trifluoromethanesulfonyl fluoride (CF₃SO₂F), comprising: performing a reaction between trifluoromethanesulfonyl chloride (CF₃SO₂Cl) and potassium fluoride in the presence of water,
wherein said performing includes: controlling the amount of the water to 0.8 mass% to 6.0 mass% based on 100 mass% of the potassium fluoride; controlling the amount of the potassium fluoride to 1.5 to 6 moles per 1 mole of the trifluoromethanesulfonyl chloride; and controlling the temperature of the reaction to 20 to 70°C.

## Patentansprüche

1. Verfahren zum Herstellen von Trifluormethansulfonylfluorid (CF₃SO₂F), welches umfasst: Durchführen einer Reaktion von Trifluormethansulfonylchlorid (CF₃SO₂Cl) mit einem Metallfluorid in der Gegenwart von Wasser,
wobei die Menge des Wassers in einem Bereich von 0,6 Massen-% und 10,0 Massen-% bezogen auf 100 Massen-% des Metallfluorids beträgt.

2. Verfahren nach Anspruch 1, wobei das Metallfluorid wenigstens eines ausgewählt aus der Gruppe bestehend aus Lithiumfluorid, Natriumfluorid, Kaliumfluorid, Rubidiumfluorid und Cäsiumfluorid ist.

3. Verfahren nach Anspruch 1 oder 2, wobei die Menge des Metallfluorids 1 bis 10 Mole pro 1 Mol des Trifluormethansulfonylchlorids beträgt.

4. Verfahren nach einem der Ansprüche 1 bis 3, wobei die Reaktionstemperatur 20°C oder höher beträgt.

5. Verfahren nach einem der Ansprüche 1 bis 4, wobei die Reaktionstemperatur 40 bis 50°C beträgt.

6. Verfahren nach einem der Ansprüche 1 bis 5, wobei die Menge des Wassers in einem Bereich zwischen 1,0 Massen-% und 3,0 Massen-% bezogen auf 100 Massen-% beträgt.

7. Verfahren nach einem der Ansprüche 1 bis 6, wobei die Reaktion durch Zugabe von Trifluormethansulfonylchlorid zu einem Hydrat des Metallfluorids durchgeführt wird.

8. Verfahren nach einem der Ansprüche 1 bis 7, wobei die Reaktion bei 20 bis 70°C unter Druckbedingungen durchgeführt wird.

9. Verfahren nach einem der Ansprüche 1 bis 8, wobei das Metallfluorid wenigstens eines ausgewählt aus der Gruppe bestehend aus Natriumfluorid, Kaliumfluorid und Cäsiumfluorid ist.

10. Verfahren zum Herstellen von Trifluormethansulfonylfluorid (CF₃SO₂F), welches umfasst: Durchführen einer Reaktion zwischen Trifluormethansulfonylchlorid (CF₃SO₂Cl) und Kaliumfluorid in der Gegenwart von Wasser,
wobei das Durchführen umfasst: Steuern der Menge an Wasser auf 0,8 Massen-% bis 6,0 Massen-% bezogen auf 100 Massen-% des Kaliumfluorids, Steuern der Menge an Kaliumfluorid auf zwischen 1,5 und 6 Mole pro 1 Mol des Trifluormethansulfonylchlorids und Steuern der Reaktionstemperatur auf 20 bis 70°C.

## Revendications

1. Procédé de production de fluorure de trifluorométhanesulfonyle (CF₃SO₂F), comprenant : la réalisation d'une réaction de chlorure de trifluorométhanesulfonyle (CF₃SO₂Cl) avec un fluorure métallique en présence d'eau,
dans lequel la quantité d'eau est comprise dans la plage allant de 0,6 % en masse à 10,0 % en masse par rapport à 100 % en masse du fluorure métallique.

2. Procédé selon la revendication 1, dans lequel le fluorure métallique est au moins l'un choisi dans le groupe constitué par le fluorure de lithium, le fluorure de sodium, le fluorure de potassium, le fluorure de rubidium et le fluorure de césium.

3. Procédé selon la revendication 1 ou 2, dans lequel la quantité du fluorure métallique est de 1 à 10 moles pour 1 mole du chlorure de trifluorométhanesulfonyle.

4. Procédé selon l'une quelconque des revendications 1 à 3, dans lequel la température de la réaction est supérieure ou égale à 20 °C.

5. Procédé selon l'une quelconque des revendications 1 à 4, dans lequel la température de la réaction est de 40 à 50 °C.

6. Procédé selon l'une quelconque des revendications 1 à 5, dans lequel la quantité d'eau est comprise dans la plage allant de 1,0 % en masse à 3,0 % en masse par rapport à 100 % en masse.

7. Procédé selon l'une quelconque des revendications 1 à 6, dans lequel la réaction est réalisée en ajoutant le chlorure de trifluorométhanesulfonyle à un hydrate du fluorure métallique.

8. Procédé selon l'une quelconque des revendications 1 à 7, dans lequel la réaction est réalisée à une température de 20 à 70 °C dans des conditions sous pression.

9. Procédé selon l'une quelconque des revendications 1 à 8, dans lequel le fluorure métallique est au moins l'un choisi dans le groupe constitué par le fluorure de sodium, le fluorure de potassium et le fluorure de césium.

10. Procédé de production de fluorure de trifluorométhanesulfonyle (CF₃SO₂F), comprenant : la réalisation d'une réaction de chlorure de trifluorométhanesulfonyle (CF₃SO₂Cl) avec un fluorure métallique en présence d'eau,
dans lequel la réalisation comprend : le contrôle de la quantité d'eau à 0,8 % en masse à 6,0 % en masse par rapport à 100 % en masse du fluorure de potassium ; le contrôle de la quantité de fluorure de potassium à 1,5 à 6 moles pour 1 mole de chlorure de trifluorométhanesulfonyle ; et le contrôle de la température de la réaction à 20 à 70 °C.
